# EUROPEAN PATENT APPLICATION

(11) **EP 4 817 539 A1**
(43) Date of publication of application: **07.10.2026**
(21) Application number: 25461517.2
(22) Date of filing: 04.04.2025
(51) Int. Cl.: A61B 17/80

(54) **PLATE AND KIT FOR FIXING JAW BONE**

(71) Applicant: CHM SPOLKA Z OGRANICZONA ODPOWIEDZIALNOSCIA, 16-061 Lewickie (PL)
(72) Inventor: JAGIELAK, Maciej Jan, 01-871 Warszawa (PL); GRAB, Pawel, 02-673 Warszawa (PL); KRZYZEK, Andrzej, 15-666 Bialysto (PL); MURAWSKI, Zbigniew, 15-034 Bialystok (PL)
(74) Representative: Bury & Bury

(57) **Abstract**

A plate **(100)** for fixing fragments of the jawbone **(400),** having an elongated shape, with an inner surface **(102)** for coming into contact with the jawbone **(400)** and an opposite outer surface **(101),** containing at least two rear openings **(130),** a front opening **(140)** and a central oblong opening **(120)** extending along the plate **(100),** provided at its front part with a socket for receiving a tool, according to the invention, is characterised in that the tool receiving socket is a positioning hole **(150)** adapted to couple with the tool. The invention also relates to a kit comprising the plate and a tool.

## Description

### TECHNICAL FIELD

The invention concerns a plate for fixing fragments of jawbone, especially in orthognathic surgery, and a kit of plate with a tool for its positioning during surgery.

### BACKGROUND OF THE INVENTION

European patent EP3518818B1 discloses a plate for fixing jawbones, having elongate shape, with an inner surface intended for contacting the jawbone and an opposing outer surface. This plate is provided with rear openings, a front opening and an oblong opening extending along the plate. The plate is provided at the front with a tool receiving socket in the form of a notch adapted to support the bifurcated tip of a tool, an ancillary device adapted for pressing. When installing the plate, the surgeon positions the plate on the two fragments of the jawbone to be fixed, earlier separated by an osteotomy line. Screws are inserted through the rear openings of the plate into the rear part of the jawbone to be fixed. The bifurcated tip of the tool is inserted into the incision and used to position the fragments of the jawbone to be fixed. A screw is then screwed into the second fragment of the jawbone through the longitudinal opening, followed by screwing the screw into the front opening of the plate.

### PROBLEM SOLVED

The aim of the invention is to solve the problem of the precise positioning of the fixing fragments of the jawbone in relation to each other and to provide the operator with greater freedom in using the tool. In addition, the invention reduces the risk of injuring the patient with the tool.

### SUMMARY OF THE INVENTION

A plate for fixing fragments of a jawbone, having an elongated shape, with an inner surface for contacting the jawbone and an opposite outer surface, having at least two rear openings, a front opening and a central oblong opening extending along the plate, provided in its front part with a tool receiving socket, according to the invention, is specific in that the tool receiving socket is a positioning hole adapted to be coupled with the tool. This allows the plate to be not only pushed but also pulled using the tool.

Preferably, the positioning hole is bean shaped, in its middle part has a widened area and is provided with an internal rim.

Preferably, the front part of the plate with the positioning hole and the back part of the plate with the rear openings bend towards the inner surface of the plate.

Preferably, the plate has three rear openings.

Preferably, the plate has by the central oblong opening a scale with a marked bone cutting line.

Preferably, the plate has by the central oblong opening a scale with a marked bone cutting line.

The invention also comprises a kit comprising the plate according to the invention and a tool having a tip adapted to couple with the positioning hole.

### ADVANTAGEOUS EFFECTS OF THE INVENTION

The disclosed plate construction reduces the risk of damage to the patient's tissue during plate positioning while providing greater plate mobility, allowing it to be not only pushed but also pulled. The plate itself has a shape with more rounded edges than the tool guided solutions known in the art, and consequently causes less tissue damage. The positioning hole, after placing the plate and the bone fragments to be fixed, is used to screw the plate in place, which improves the distribution of forces on the plate surface and promotes integration with the bone. The tool has a shape with rounded edges, safe for tissue and interacts with the plate with minimal contact with the patient's tissues.

### BRIEF DESCRIPTION OF THE DRAWINGS

The invention is disclosed in the embodiments discussed below with reference to the drawings, wherein Fig. 1 shows a plate according to an embodiment of the invention as viewed from the outer side, Fig. 2 shows a plate according to an embodiment as viewed from the inner side, Fig. 3 shows the plate according to the embodiment of the invention in a front view, Fig. 4 shows the plate according to the embodiment of the invention in a rear view, Fig. 5 shows the plate according to the embodiment of the invention in a top view, Fig. 6 shows the tool for positioning the plate according to the embodiment of the invention, Fig. 7 shows the plate according to an embodiment of the invention with screws and with the tool coupled, Fig. 8 shows the coupling location in enlarged view, Fig. 9 shows the plate according to an embodiment of the invention in process of fixing to the jawbone, and Fig. 10 shows the plate according to the embodiment of the invention fixed to the jawbone.

### DESCRIPTION OF EMBODIMENTS

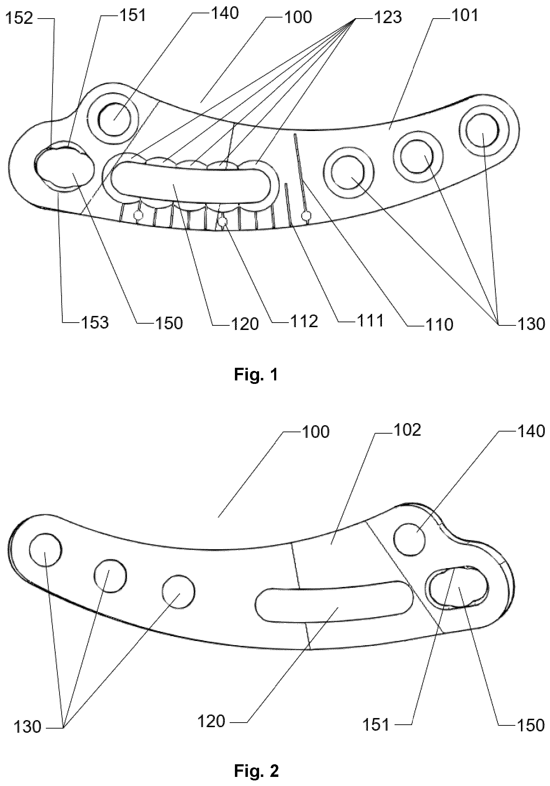

Plate **100** according to the example of embodiment of the invention is used to fix fragments **401, 402** of the jawbone (mandible) **400.** In particular, the plate is intended for osteotomy. Plate **100** is shown in an external view in Fig. 1. Plate **100** has an elongated shape, with an inner surface **102** intended to contact the jawbone **400** and an opposite outer surface **101.** The plate **100** according to the embodiment has three rear openings **130,** adapted to connect it to the rear fragment **401** of the jawbone **400.** In this description, the front and rear directions denote the front and rear of the patient with the plate mounted. Similarly, top and bottom refer to the orientation of the plate mounted on a patient holding head upright. The plates are manufactured in left and right variants. This embodiment is discussed with reference to the plate in the left variant, adapted to connect the jawbone on the left side of the patient.

As a general rule, two rear openings are sufficient, but a third opening provides greater connection reliability and reduces the risk of damage when positioning parts **401** and **402** of jawbone **400** relative to each other.

Plate **100** is additionally provided with a front opening **140** and a central oblong opening **120** extending along plate **100.** In its front part, plate **100** is provided with a socket for receiving tool **360.**

The bottom edge of the plate is bent in the plane of the outer surface. In this example, the radius of curvature is 35 mm. The plate can be made in various sizes to fit the size of the patient's jawbone, as well as in right and left variants.

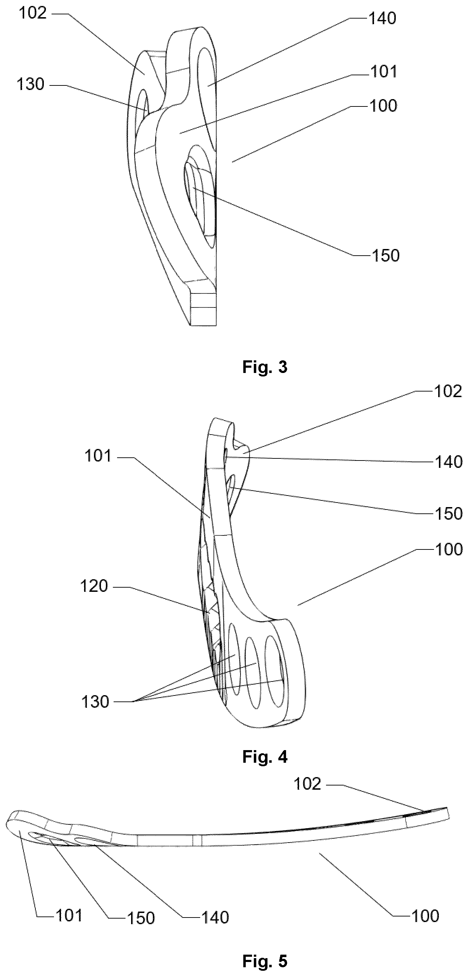

In this embodiment, the front part of plate **100** with positioning hole **150** and the rear part of plate **100** with rear openings **130** bend towards the inner surface **102.** Thus, the plate has an arched shape that fits well to the jawbone **400.** This is shown in Fig. 3, which shows plate **100** in front view, Fig. 4, which shows plate **100** in rear view, and Fig. 5, which shows a top view. As a result, plate **100** fits well over the jawbone and integrates much better therewith. Studies have shown that for most patients, a plate can be fitted with a front curvature radius of 5 mm to 15 mm and a rear curvature radius of 60 mm to 100 mm. In this embodiment, the radius of curvature of the front part of the plate is 10 mm and the radius of curvature of the rear part of the plate is 80 mm. With these values, the plate is quite universally adaptable. It should be noted that in other embodiments, straight plates can be used in accordance with the invention, but the bends discussed improve the fit of the plate to the bone, which results in a reliable connection and reduces the risks to which the patient is exposed.

The tool receiving socket is a positioning hole **150** adapted for coupling with the tool. The coupling can be achieved in various ways and the positioning hole can have different shapes and cross-sections, in particular it can have the shape of a slot. In this embodiment, the positioning hole **150** is essentially bean-shaped and has a widened area **151** in its middle part and is provided with an internal rim **152.** The tip **361** of the tool can be inserted through the widened area **151** of the hole **150** and hooked into the rim **152.**

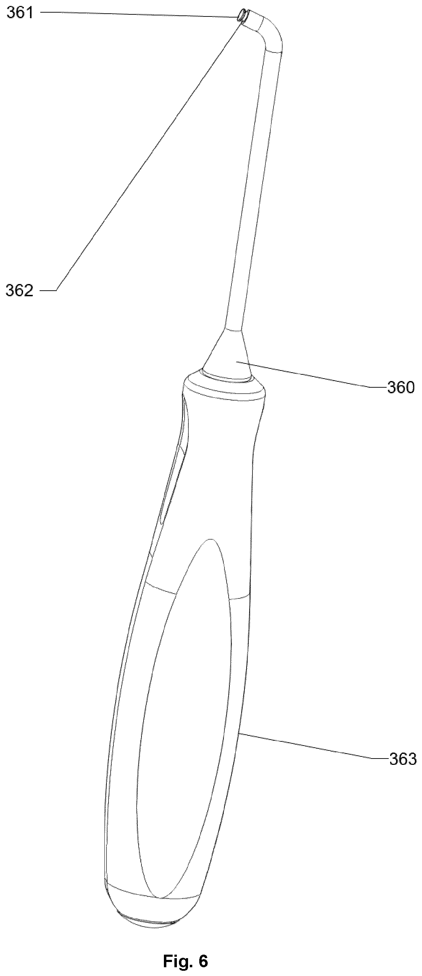

The plate positioning tool **360** is shown in Fig. 6. The tool **360** is provided with a notch **362** at the end **361.** The notch **362** fits into the rim **152.**

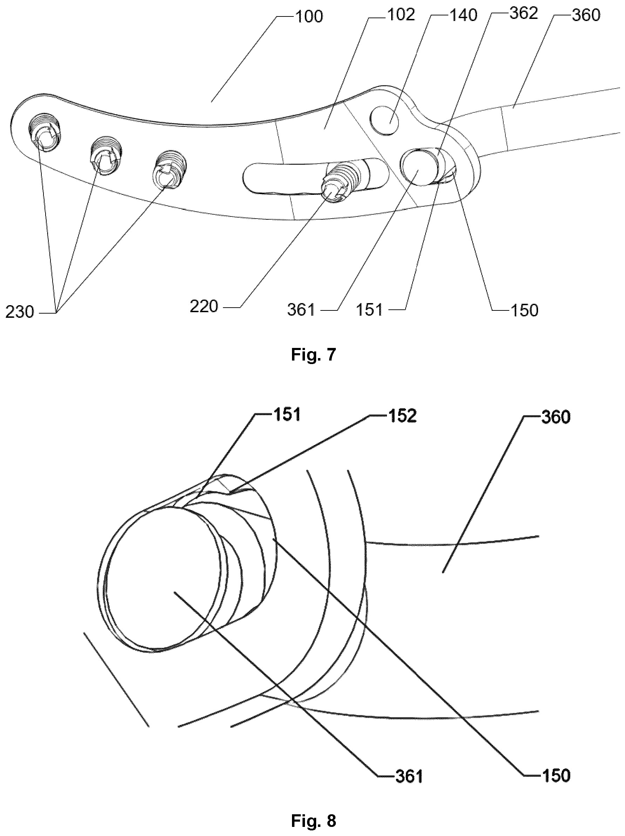

The plate **100** from the inner side with screws **230** in the rear openings **130** and screw **220** in the central oblong opening **120** and with tool **360** with tip **361** placed in the positioning hole **150** is shown in Fig. 7. After placing the tip **361** in the hole **150,** the rim **152** enters the notch **362,** as shown in the enlarged view in Fig. 8.

The plates according to the invention can be manufactured in various sizes and in left and right versions. In this embodiment, a plate with a thickness of 0.8 mm is illustrated. Plates having thickness within a range 0,6 mm to 2,0 mm were successfully tested. The radius of curvature of the plate in the vertical plane is 35 mm. It has been established through testing that, in 90 percent of patients, a plate with a radius of curvature within the range of 20 mm to 50 mm can be effectively fitted.

As can be seen in Figs. 1, 9 and 10, on the plate according to the embodiment, a scale has been applied to allow control of the correction value. The scale used has a graduation with a mark every 1 mm and an auxiliary dot every 5 mm.

The invention was tested with various positioning holes. In general, openings with a width of 2 mm to 3.4 mm worked well. In this example, a opening with a width of 2.6 mm was used. This made it easy to adapt the tool passing through the 2.6 mm wide widened area, but hooking into the narrower parts of the opening, where the clearance width through the rim is 2 mm.

Additionally, the positioning hole on the outer side **101** of plate **100** has a chamfer **153** in the centre. This allows a screw to be inserted into the positioning hole after plate **100** has been attached to the jawbone. This solution allows better use of the plate surface and more secure attachment.

The use of a positioning hole instead of an incision also gives the plate a more rounded shape, which reduces the risk of injury to the patient's tissues.

The rear openings **130** and the front opening **140** in the present embodiment have a diameter of 2.1 mm. In general, diameters ranging from 1.5 mm to 2.5 mm have worked well. Due to the possibility of using the same screws, it is advisable to use an oblong central opening **120** with the same width as the diameters of the rear and front openings. For operational reasons and due to the variation in the size of patients' jawbones, it is best if the length of the central opening **120** ranges from 5 mm to 25 mm. In this embodiment, an 8 mm long opening was used. A **123** chamfer was also used to increase the stability of the connection after final tightening. Preferably, the difference between the width of the positioning hole and the other openings is so small that the same screws can be used, which reduces the risk of mistakes.

In this embodiment, the plate is made of pure titanium, but in other embodiments, titanium alloys have been used successfully. Pure titanium is more ductile, which is beneficial for manufacturing a plate with such a complex shape, while titanium alloys are more durable.

Plate **100** in this example has a scale applied to its outer side **101** at the centre opening **120** to ease the positioning of fragments **401, 402** of the jawbone **400** relative to each other. The scale is marked with a cutting line **110** for osteotomy and lines **111** every 1 mm and, for assistance, dots **112** every 5 mm. The scale facilitates the precise positioning of fragments **401, 402** in accordance with the planned surgical procedure. Different scales can be used, but experiments have shown that a 1 mm scale is sufficiently accurate, and the additional marking of lines 5 mm apart reduces the risk of mistakes. In surgical conditions, the dots **112** proved easier to distinguish from the bold lines.

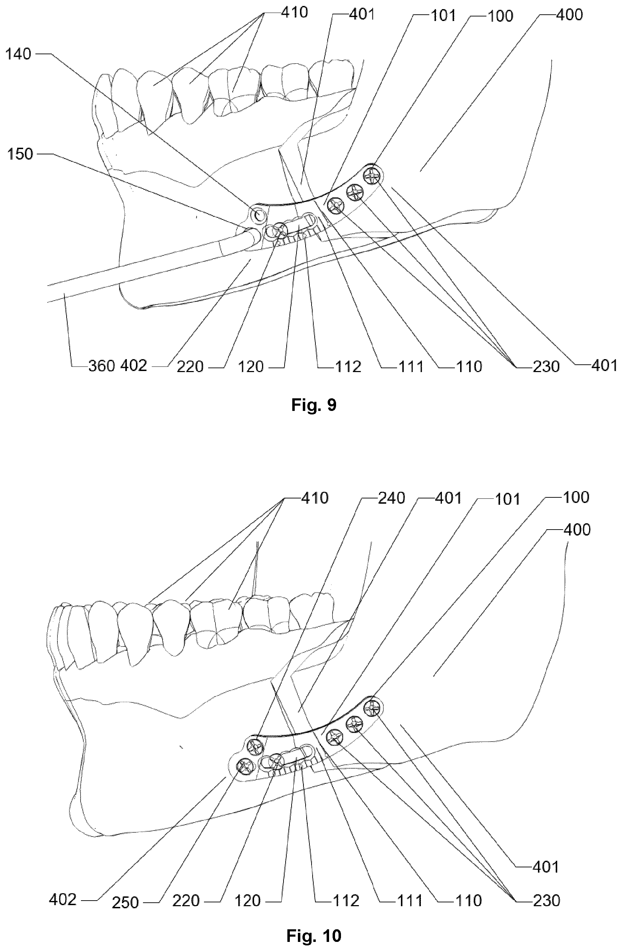

Plate **100** on the jawbone **400** during positioning of fragments **401** and **402** during osteotomy is shown in Fig. 9. Screws **230** placed in rear openings **130** are tightened. Thus, plate **100** is rigidly connected to the rear fragment **401** of the jawbone. It is positioned so that line **110** corresponds to the end of the rear fragment **401** of the jawbone **400.** Screw **220** in the oblong central opening **120** is not tightened, so that plate **100** can move relative to the front fragment **402** of the jawbone. Tool **360** is coupled with the positioning hole **150** of plate **100,** so that it can be used to position the fragments of the jawbone **400** relative to each other using the scale **110, 111, 112,** moving them apart to the desired distance. Once the correct position has been set, screw **220** is tightened. Next, screw **240** is screwed into the front opening **140** and screw **250** is additionally screwed into the positioning hole **150,** achieving a secure and reliable connection, as shown in Fig. 10.

Coupling the tool to the plate through the positioning hole **150** has the advantage over the open slot solution known from the prior art in that it allows front-to-back movement and also allows the use of a rounded tool, which reduces the risk of unnecessary injury to the patient. The combination of the scale with the tool coupling system enabling two-way movement of the plate **100** has proven to be unexpectedly effective, greatly facilitating the precision of the procedure.

### INDUSTRIAL APPLICABILITY

Disclosed plate is particularly suitable for surgical treatment and shaping of the jawbone but can also be used successfully in the treatment of fractures.

## Claims

1. A plate **(100)** for fixing fragments of a jawbone **(400),**
having an elongated shape, with an inner surface **(102)** for contacting the jawbone **(400)** and an opposite outer surface **(101),**
having at least two rear openings **(130),** a front opening **(140)** and a central oblong opening **(120)** extending along the plate **(100),**
provided at its front part with a socket for receiving a tool,
**characterized in that**
the socket for receiving the tool is a positioning hole **(150)** adapted to couple with the tool.

2. Plate according to claim 1, wherein the positioning hole **(150)**
is bean shaped and
has a widened area **(151)** in its middle part and
is provided with an internal rim **(152).**

3. Plate **(100)** according to claim 1 and 2, wherein the part of the plate with the positioning hole **(150)** and the part of the plate **(100)** with the rear openings **(130)** are bent towards the inner surface **(102).**

4. Plate **(100)** according to claim 1 or 2 or 3, having three rear openings **(130).**

5. Plate **(100)** according to any of claims 1 to 4, having on the central longitudinal opening **(120)** a marked scale **(110, 111, 112).**

6. Plate **(100)** according to any of claims 1 to 5, having at the central longitudinal opening **(120)** a marked scale **(111, 112)** with an indicated bone cutting line **(110).**

7. A kit comprising a plate as defined in any of claims 1 to 6 and a tool **(360)** having a tip **(361)** adapted to couple with the positioning hole **(150).**
